# EUROPEAN PATENT APPLICATION

(11) **EP 1 531 000 A1**
(43) Date of publication of application: **18.05.2005**
(21) Application number: 04256803.0
(22) Date of filing: 04.11.2004
(51) Int. Cl.: B01J 31/10

(54) **Catalytic reactions using thermally stable resins**

(30) Priority: 13.11.2003 US 519687 P
(71) Applicant: Rohm and Haas Company, Philadelphia PA 19106-2399 (US)
(72) Inventor: Ramprasad, Dorai, Allentown Pennsylvania 18104 (US); Collin, Jennifer Reichl, Devon Pennsylvania 19333 (US)
(74) Representative: Buckley, Guy Julian

(57) **Abstract**

The present invention provides a system, method and catalyst for a variety of reactions, e.g. aldolization, etherification, oligomerization, dimerization, dehydration and condensation. The invention also includes systems for making various end products, and end products made using the systems and methods described herein.

## Description

This invention relates to a method of, and system and catalyst for olefin dimerization, etherification, and condensation. The invention also provides for a method of making the catalyst capable of olefin oligomerization, aldolization, dehydration, etherification, and condensation, and a catalyst composition.

Sulfonated polystyrene resins crosslinked with divinyl benzene have been used as catalysts for a variety of reactions. Olefin oligomerization has been used to make dimers and oligomers. By way of example, the dimerization of isobutylene to isooctene followed by hydrogenation produces isooctane, an important additive for gasoline. Other important reactions are etherifications. Etherifications are to prepare oxygenates that are important additives for gasoline or diesel. For example, isobutylene is reacted with methanol or ethanol to give MTBE(methyl tert-butyl ether) and ETBE(ethyl tert-butyl ether) respectively. Similarly, isoamylene is reacted with methanol to give TAME(tert-amyl methyl ether). Another reaction is the dehydration of 1-pentanol to di-n-pentyl ether which is an additive for diesel. Other important reactions include the use of catalysts in aldolizations. For example, acetone is dimerized to get diacetone alcohol. Then it is dehydrated to mesityl oxide followed by hydrogenation to make the important chemical MIBK(methyl isobutyl ketone). Other important products from aldolization reactions are pentaerythritol. Other reactions are condensations such as the reaction of phenol and acetone to give bis-phenol A an important precursor to polycarbonates. It is an object of the invention to provide catalysts that can be used in the variety of reactions described above.

No high temperature stable resins have been used as catalysts for the reactions described above. There are significant problems with the use of conventional resins at high temperatures. The use of elevated temperatures has the significant drawback of a resulting loss of SO₃H groups in the catalyst. This reduces the catalyst activity and corrodes a reactor. There exists a need for thermally stable catalysts capable of catalyzing the above reactions at high temperatures. This need is filled by the present invention.

Another major problem of all of the attempts in the prior art to prepare effective catalysts that can be used in the reactions described above and below is corrosion of reactors and piping, and the need for very long reaction times. Conventional chlorinated polymers split off HCl in addition to sulfuric acid during use, and lead to corrosion of stainless steel reactors. US Patent No. 4,705,808 generally relates to a process for pretreatment of a catalyst with deionized water for 400 hours in the absence of oxygen or metal ion at 100-150 degrees C under pressure. This art still has the drawback of requiring significant reaction times. There exists a need in the art for a thermally stable catalyst, method and system that solves the corrosion problem and provides for greatly reduced reaction times.

There exists a significant need in the art for methods of aldolization, etherification, oligomerization, dehydration and condensation, and for catalysts that are used for aldolization, etherification, oligomerization, dehydration and condensation reactions that are thermally stable and provide improved performance characteristics.

### STATEMENT OF THE INVENTION

In one aspect, the present invention relates to a method of aldolization, etherification, oligomerization, dehydration and/or condensation using a catalyst that includes a styrene DVB copolymer, and/or a DVB and EVB copolymer. The method includes using a base treated, sulfonated, halogenated and acid regenerated thermally stable catalyst.

In yet a further aspect, the invention provides an improved styrene DVB resin catalyst comprising 4-chlorostyrene aromatic groups and a polymer backbone. At least one of the aromatic groups has a chlorine in a styrenic para-position, and optional other chlorines thereon. The polymer backbone is substantially free of leachable chlorine. The catalyst includes a halogenated DVB moiety. In one variant, the aromatic rings are oleum sulfonated. In yet another variant, the styrene DVB resin further comprises sulfone bridges. Another variant includes a 4-halo (F,Br, and/or I) styrene.

### DETAILED DESCRIPTION

The present invention relates to a method of aldolization, etherification, oligomerization, dehydration and condensation using a catalyst that includes a styrene DVB copolymer and/or DVB/EVB copolymer. The method includes using a base treated, sulfonated, halogenated and acid regenerated thermally stable catalyst. Exemplary reactions include dehydration of pentanol to di-n-pentyl ether, acetone conversion to mesityl oxide, isobutylene dimerization and the synthesis of Bisphenol-A ("BPA"). Use of the catalysts, methods and systems described herein provides unexpected performance improvements in aldolization, etherification, oligomerization, dehydration and condensation.

In another aspect, the invention provides a method of making a thermally stable catalyst used for aldolization, etherification, oligomerization, dehydration and condensation reactions. The method includes base treating a sulfonated and halogenated copolymer to obtain a base treated copolymer, and regenerating the base treated copolymer with an acid. In another variant, the method of making a thermally stable catalyst for aldolization, etherification, oligomerization, dehydration and condensation includes base treating a halogenated copolymer to obtain a base treated copolymer, and sulfonating the base treated copolymer.

In yet another variant, leachable chlorine is removed from a polymer backbone of the catalyst by the base treatment. Manufacture of the catalyst described herein has the advantage of reducing the level of corrosion of equipment used for aldolization, etherification, oligomerization, dehydration and condensation reactions versus conventional catalysts since there is significantly reduced leachable chlorine on the polymer backbone of the catalyst described herein. It is appreciated that significant cost savings are achieved and equipment longevity is increased through the use of the method, catalyst, and systems that use the method and catalyst described herein. Moreover, in one variant of the invention a polymer backbone is substantially free of leachable chlorine when there are no or very limited corrosion effects on equipment and/or prolonged life of the equipment as a result of use of the catalyst described herein.

In yet a further variant, the method of making a thermally stable catalyst for use in aldolization, etherification, oligomerization, dehydration and condensation reactions includes base treating a sulfonated and halogenated copolymer to obtain a base treated copolymer, and regenerating the base treated copolymer with an acid. The method of making a thermally stable catalyst for aldolization,etherification, oligomerization, dehydration and condensation optionally includes chlorinating a styrene DVB copolymer, base treating the halogenated copolymer, and sulfonating the halogenated and base treated catalyst. The styrene DVB copolymer is a polysulfonated copolymer in another variant of the invention.

In yet a further aspect, the invention provides an improved styrene DVB resin catalyst comprising aromatic groups having more than one SO₃H moiety and a polymer backbone. The improvement includes halogenated aromatic groups. The polymer backbone is substantially free of leachable chlorine. The catalyst is capable of catalyzing aldolization, etherification, oligomerization, dehydration and condensation reactions. In one variant, the leachable chlorine is at least an order of magnitude less than the leachable chlorine of a catalyst not base treated. In yet a further variant, the polymer backbone of the catalyst is free of leachable chlorine or other detrimental halogens.

In yet a further aspect, the invention provides an improved styrene DVB resin catalyst comprising 4-chlorostyrene or any 4-halostyrene aromatic groups and a polymer backbone. At least one of the aromatic groups has a chlorine or other halogen in a styrenic para-position, and optional other chlorines or halogens thereon. The polymer backbone is substantially free of leachable chlorine or other halogens. The catalyst includes a halogenated DVB moiety. In one variant, the aromatic rings are oleum sulfonated. In yet another variant, the styrene DVB resin further comprises sulfone bridges.

In yet a further aspect, the invention provides a method of reducing the compressibility of a styrene DVB monosulfonated catalyst. The method includes increasing the number of chlorine on aromatic rings of the catalyst as compared to unhalogenated catalysts. In yet a further variant of the invention, the method of aldolization, etherification, oligomerization, dehydration and condensation utilizes a base treated, sulfonated, chlorinated and acid regenerated thermally stable catalyst.

Variants of the invention relate to preparation of resins of high thermal stability with low leaching of chlorine which are used as catalysts with high activity for aldolization, etherification, oligomerization, dehydration and condensation, e.g. propene and/or butene hydration. Several types of catalytic resins were prepared according to the reaction schemes shown below.

The first reaction scheme for making a catalyst of the present invention involves the following process: Styrene DVB copolymer --→ Sulfonate -→ chlorinate (or halogenate) -→ base treat --→ regenerate with acid. The chlorination (or halogenation) step incorporates chlorine (or other halogen) in the aromatic ring as well as the aliphatic backbone of the polymer. It is the chlorine (or other halogen) on the backbone that can undesireably leach of slowly as HCl (or other corrosive compound) in an aldolization,etherification, oligomerization, dehydration and condensation process. Where this happens, accelerated equipment corrosion occurs resulting in undesirable down time and equipment replacement costs. Heating the polymer in a basic solution results in, for example, the leachable chlorine being removed. Moreover, this process is carried out in the space of a several hours in contrast to the art process which takes 10 days to carry out, e.g. U.S. Patent No. 4,705,808. It is appreciated that the time needed to manufacture a catalyst of the invention is greatly reduced to the range of several hours, e.g. 1-10 hours in one variant of the invention.

The final polymer is created using the process that has a higher acid site density than that created by prior methodologies. The current process does not have the drawback of the known processes since there is no extensive de-sulfonation in addition to chlorine removal. Consequently, a catalyst created by the process used herein has the advantage of lower de-sulfonation than catalysts produced by conventional processes. This results in higher catalytic activity since more active sites remain on the catalyst after preparation and after catalyst use than with conventional catalysts.

Scheme 1 was used to chlorinate Amberlyst 39, a commercially available sulfonated styrene DVB(7%) co-polymer from Rohm & Haas Company. Other suitable resins could also be used herein. After refluxing with a 2N sodium hydroxide solution for 22 hours, followed by regeneration with hydrochloric acid, a new resin (A) was obtained. The hydrolytic stability of this resin was tested at 200°C for 24 hours. Table 1 shows that Resin A loses 9.5% of its sulfonic acid groups versus 33.4% lost by resin **F** which is a conventional chlorinated resin containing 12% DVB. Additionally, the percentage chlorine detected in solution is much less for resin **A**. We also subjected resin **F** to a base treatment to get resin **B** which has somewhat improved stability and lower chlorine leaching than resin **F**. Interestingly, resin **F** was also post treated using the process described in U.S. Patent No. 4,705,808 to get resin **C.** Table 1 indicates that resin **B** has a higher acid capacity and is different from resin **C** in its properties and performance characteristics. The variant of the invention of scheme 1 involves a chlorination step followed by base treatment prior to the sulfonation. Additionally, the base treatment is performed in any other solvent or mixtures of solvents in other variants of the invention. The optimum conditions are resin specific, and can readily be determined empirically. In the examples described, we have described the use of a 50% NaOH solution for 4 hrs at 130 degrees C.

Scheme 2 is also used to obtain a catalyst of the present invention:
Styrene DVB copolymer --→ polysulfonate using oleum -→ chlorinate (or otherwise halogenate)-→ base treat --→ regenerate with acid.

Oleum sulfonation of a styrene DVB co-polymer results in a polysulfonated polymer (one that has greater than one SO₃H group per ring). It is appreciated that the resin created by the process is both a polysulfonated and a chlorinated resin in one variant of the invention. Amberlyst 36 is a polysulfonated resin with 12% DVB. This resin was chlorinated and base treated as in Scheme 2 to get resin D. The thermal stability results of resin D (see Table 1) show that it has high acid capacity, good stability and low chlorine leaching.

Scheme 3 is also used to obtain a catalyst of the present invention: 4-chlorostyrene DVB copolymer -→ sulfonate using oleum -→ chlorinate (or otherwise halogenate)-→ base treatment -→ regenerate with acid. A monomer is prepared by British Patent 1,393,594. A halogenated monomer such as chlorostyrene with a crosslinker such as divinylbenzene is used, thereafter one sulfonates the polymer using chlorosulfonic acid. This polymer is still unstable at high temperatures because the DVB part is not deactivated by a halogen atom. In this variant of the invention, this polymer is chlorinated even further creating very desirable catalytic properties as well as solving the temperature stability problem by having high termperature stability. While not being bound by theory, we hypothesize that a chlorination of the DVB creates a very stable resin since all the SO₃H groups on the chlorinated resin will be in a meta position.

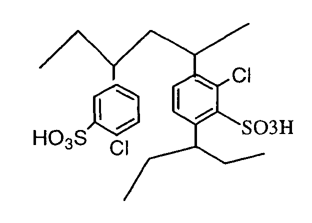

In another variant of the invention, a copolymer of 4-chlorostyrene with 12% DVB was prepared and then monosulfonated using oleum as the sulfonating agent for relatively short reaction times. This is a substantial improvement on the procedure described in British Patent 1,393,594. The resulting resin has sulfone bridges which increase thermal stability. The resin was then further chlorinated and then base treated to remove leachable chlorine and after acid regeneration yielded resin **E**. The thermal stability of this resin and the low chlorine leaching coupled with the high acid capacity provides the resin with important catalytic properties and unexpected performance characteristics. Scheme 3 can therefore be used to prepare a variety of styrene DVB polymers that are fully halogenated and have SO₃H groups in a meta position. This method is also used to prepare polysulfonated chlorinated resins as in Scheme 2 by varying the sulfonation conditions. In addition to this, the chlorination of the copolymer and base treatment is performed prior to the sulfonation step.

It is further appreciated that the steps described in the various schemes mentioned above are combined in various permutations and combinations such that the desired catalytic properties of the catalyst are obtained herein.

**TABLE 1**

| RESIN | %Cl %S | MmolSO3H/ g | Mmol SO3H/g after 200C, 24hours | % loss in acid sites | % chlorine in liquid after 200C, 24 hours |
|---|---|---|---|---|---|
| **A** | 26.78,8.62 | 2.75 | 2.49 | 9.45 | 0.2 |
| **B** | 19.62, 10.24 | 3.31 | 2.50 | 24.60 | 0.19 |
| **F** | 22.00 | 3.14 | 2.10 | 33.40 | 1.34 |
| **C** | 19.30 | 3.06 | 2.12 | 30.40 | 0.08 |
| **D** | 10.04, 16.69 | 4.78 | 3.79 | 20.70 | 0.11 |
| **E** | 15.96, 13.95 | 4.16 | 3.52 | 15.34 | 0.12 |

### COMPRESSIBILITY OF RESINS

Chlorination (or other halogenation) of a monosulfonated styrene DVB resin reduces its compressibility. The change in the compressibility depends on the percent DVB in resin and on the degree of chlorination or other halogenation. The results are shown below.

| RESIN | COMPRESSIBILITY cm/m |
|---|---|
| Styrene 12% DVB copolymer monosulfonated | 4.03 |
| Styrene 12% DVB copolymer chlorinated to 20% by weight | 3.03 |
| Styrene 7% DVB copolymer monosulfonated | 7.23 |
| Styrene 7% DVB copolymer monosulfonated and chlorinated to 27% by weight | 4.39 |

### Example 1

Polymer synthesis: Styrene DVB co-polymers used to make catalysts of the present invention are commercially available in sulfonated forms. For example, Amberlyst 39 (commercially available from Rohm and Haas Company) was chlorinated to make resin **A** using Scheme 1. Similarly, Amberlyst 16 was used to prepare resin **F**. Treatment of resin **F** with water at 150 degrees **C** for ten days according to US patent no. 4,705,808 produced resin **C**. Amberlyst 36 was chlorinated to make resin **D** according to Scheme 2. For resin **E**, the substrate 4-chlorostyrene co-polymer used to make a catalyst of the present invention was according to art procedures described in British Patent 1,393,594. The sulfonation was done using oleum at 110°C for 1 hour - 100g of polymer required 1000g of oleum.

### Example 2

Chlorination of polymer: The following procedure is representative for all examples and chlorinations. However, it is appreciated that other chlorination procedures can be used to obtain the properties of the catalyst described herein: In a two liter glass reactor connected to a water circulation bath was placed 490 ml of wet Amberlyst 36. To this was added 1180 g of water, and after stirring the water circulation bath was set to 35°C. The system was purged out for ten minutes with nitrogen and then chlorine was introduced into the reactor at 15 psig. The chlorine feed rate is a function of reaction rate and was monitored by measuring weight loss of lecture bottle. The percent HCl in the liquid was a measure of how much chlorine was on the resin since each Cl atom on the resin produces one HCl which dissolves in the water. This serves as a tool to gauge how much chlorine was on the resin. Aliquots of solution were removed and titrated with base to measure percentage HCl in solution. After 15 hours, the percentage HCl was found to be 2 percent. The reaction was stopped and the resin was washed with several rinses of DI water then sent for analysis. Found Cl : 11.87%, S: 16.66%

### Example 3

The following experiment illustrates that base treatment removes leachable chlorine: 75 grams of wet resin F was mixed with 220ml of 50% NaOH and heated for 4 hours at 130C. The resin was filtered and the filtrate plus washes were weighed. A 10 g sample of the filtrate was treated with nitric acid to a pH of 2, then titrated with silver nitrate. Based on this result the amount of chlorine in 10gm of filtrate was normalized to the overall weight of filtrate plus washes. The amount of leachable chlorine in 75 g of wet resin was determined as 1.46g. The same experiment when repeated at room temperature for ten minutes gave only 0.47g of chlorine showing that the heat treatment with base removes additional chlorine from the polymer.

### Example 4

The following base treatment procedure was used to make resins **A**, **B**, **D**, **E**. Approximately 275 grams of chlorinated resin (washed with 1500 ml of deionized water) was placed in a 3 neck round-bottomed flask equipped with a mechanical stirrer, water condensor and a Thermowatch™. To this was added 1192 ml of a 2N NaOH solution. The mixture was stirred and heated to reflux (103 degrees C) for a total of 22 hrs. The solution was separated from the resin and combined with a 100 ml washing of the resin. The solution was acidified with HNO₃ and then titrated with silver nitrate for chloride determination. The resin was washed 3 times with 500 ml of deionized water then transferred to a column and washed with 3 liters of water (3 hrs) and 3 liters of 4% hydrochloric acid (3hrs) and then three liters of water(3hrs).

For resin **A** - the following was found before base treatment: 29.7% Cl, 8.2% S. After base treatment, the following results were obtained: 26.78% Cl, 8.62% S. The calculated amount of chloride recovered from the resin is 6.79g which is consistent with the reported analysis.

For resin **D** - the following was found before base treatment: 11.87% Cl, 16.66% S. After base treatment, the following results were obtained: 10.04% Cl, 16.69% S.

For resin **E** - the following was found before base treatment: 18.87% Cl, 13.97% S. After base treatment, the following results were obtained: 15.96% Cl, 13.95% S.

### Example 5

The following example gives one variant of a thermal stability test procedure used herein: In a 125 ml acid digestion bomb is added 40 ml of resin and 28 ml of deionized water. The bomb was sealed and placed in a vacuum oven which was then heated to 200 degrees C. After 24 hours, the heat was turned off and the oven was cooled for 4 hours. The bomb was removed and the lid was removed in a hood. The liquid was separated from the resin and the pH was measured and the chlorine content determined by titration. The resin was washed thoroughly and its acid capacity was measured as described in example 6. The same experiment was also repeated in three smaller digestion bombs, each containing 14 ml of resin and 10 ml of water. After the thermal stability experiment the three samples were combined for analysis. Results are shown in Table 1.

### Example 6

The following procedure was a general method used to determine acid capacity of all resins. The acid capacity of resin was determined as follows: The wet resin was placed in a beaker and dried overnight at 110 degrees C. The beaker was placed in a dessicator allowed to cool and weighed. The first weight observed on the balance was recorded (allowing the resin to sit exposed to the air results in re-absorption of moisture). The weight of dry solid was recorded (typically we used between 5.5 and 6.0g). After transferring to a column the resin, approximately 300 ml of deionized water was passed through the resin for half hour. Separately, a solution of sodium nitrate was prepared by dissolving 45 g in a liter of water. This flowed through the column in 2 hours and the eluate was collected in a 1000 ml volumetric flask up to the mark. 100 ml of this sample was titrated with 0.1008 N solution of sodium hydroxide. The acid capacity was calculated as [V1*0.1008*10/W1] mmol H+ per gram of dry resin where V1 is the volume of base required for neutralization and W1 is the dry weight of resin.

### Example 7

### ALDOLIZATION

Resin A was placed in a thimble in a Soxhlet™ extractor. Acetone was refluxed to its boiling point at 56°C and passed over the resin and the diacetone alcohol was collected in the flask below. After an hour the conversion was complete.

### Example 8

### ETHERIFICATION/DEHYDRATION

5 grams of resin sample D is dried at 120°C for 3 hours and then placed with 25 ml of n-pentanol in an 100ml autoclave and stirred at 500 rpm and heated to 150C. Samples are taken at regular intervals for 6 hours to get di-n-pentyl ether with 45% conversion and 95% selectivity.

### Example 9

### Dimerization/Oligomerization

Resin sample A is tested for isobutylene dimerization as outlined in Catalysis Today vol. 65, 397 (2001). A methanol to olefin ration of 0.2 is used and passed over a fixed bed of A at 50°C and LHSV (liquid hourly space velocity) of 7h⁻¹ LHSV. A conversion of 60% is obtained with a selectivity to dimer of >90% using the resin of the present invention.

The DVB used herein is in the range of 63 to 100 percent purity. The DVB obtained commercially for use in the invention can also use alone or in combination with EVB (ethylvinylbenzene), which can also be used in combination with DVB.

Other reactions which can be catalyzed using the novel resin catalysts described herein include: a formaldehyde and alcohol reaction, a reaction to make a diol from an epoxide, a reaction to make an ether from a phenol, etherification of phenols and naphthols using olefins such as isobutylene and isoamyleine, an dimerization reaction of isobutylene to make isooctene, a dimerization/oligomerization reaction of other olefins (e.g. isoamylene, and alpha-methyl-styrene), a hydration reaction (e.g. hydration of acrolein to make hydropropanaldehyde, which is then hydrogenated to 1-3-propanediol).

## Claims

1. A method of catalyzing a reaction using a catalyst, comprising: catalyzing said reaction using a base treated, sulfonated, halogenated and acid regenerated thermally stable catalyst, said catalyst comprising a styrene DVB copolymer, or a styrene DVB/EVB copolymer, or a combination thereof.

2. The method of claim 1 in which said reaction is selected from the group consisting of an aldolization reaction, an etherification reaction, an oligomerization reaction, a dehydration reaction, and a condensation reactions.

3. The method of claim 1 in which said reaction is selected from a formaldehyde and alcohol reaction, a reaction to make a diol from an epoxide, a reaction to make an ether from a phenol, etherification of a phenol and naphthol using an olefins, an oligomerization reaction, a dimerization reaction of an olefin, and a hydration reaction.

4. The method of claim 1 in which said reaction is selected from the group consisting of a reaction to make MTBE, a reaction to make ETBE, a reaction to make TAME, a reaction to make di-n-pentyl ether, a reaction to a diacetone alcohol, a reaction to make MIBK, a reaction to make pentaerythritol, and a reaction to make bis-phenol A.

5. A system for catalyzing a reaction utilizing the method of claim 1.

6. The system of claim 5 in which said system comprises an MTBE manufacturing system, ETBE manufacturing system, TAME manufacturing system, di-n-pentyl ether manufacturing system.

7. The method of claim 1 in which said catalyst comprises aromatic rings that are oleum sulfonated.

8. A product made using the method of claim 1.

9. The product made using a system of claim 5.
